# EUROPÄISCHE PATENTANMELDUNG

(11) **EP 0 665 026 A2**
(43) Veröffentlichungstag der Anmeldung: **02.08.1995**
(21) Anmeldenummer: 94116957.5
(22) Anmeldetag: 27.10.1994
(51) Int. Cl.: A61M 1/16, A61J 1/00

(54) **Dialysevorrichtung**

(30) Priorität: 30.12.1993 DE 9320151 U
(71) Anmelder: B. BRAUN MELSUNGEN AG, D-34212 Melsungen (DE)
(72) Erfinder: von der Haar, Friedrich, D-34212 Melsungen (DE); Meyer, Günther, Dipl.-Ing., D-34212 Melsungen (DE); Rath, Dieter, D-34212 Melsungen (DE); Schacht, Bodo, D-34323 Malsfeld (DE)
(74) Vertreter: Selting, Günther, Dipl.-Ing.

(57) **Zusammenfassung**

Die Dialysevorrichtung weist einen flexiblen Folienbeutel (10) auf, dessen Zulaufschlauch (22) mit dem Wasserlieferanschluß eines Dialysegeräts verbunden wird und dessen Ablaufschlauch (13) mit dem Konzentrat-Aufnahmeanschluß des Dialysegeräts verbunden wird. Der Folienbeutel (10) enthält eine wasserlösliche trockene Substanz (26) und er wird von Wasser durchströmt, wodurch ein für die Dialyse erforderliches Konzentrat erzeugt wird.

## Beschreibung

Die Erfindung betrifft eine Dialysevorrichtung mit einem Dialysegerät, das an einen Behälter zur Lieferung eines Dialysekonzentrats oder einer Desinfektionsmittellösung angeschlossen ist.

Dialysevorrichtungen weisen eine Dialysatleitung auf, die zu dem Dialysator für die Blutwäsche eines Patienten führt. Die Dialysatleitung ist an einen Wasserbehälter und an mindestens einen Konzentratbehälter, der flüssiges Konzentrat enthält, angeschlossen, wobei das flüssige Konzentrat dosiert in die Dialysatleitung injiziert wird.

Es ist bekannt, anstelle eines Konzentratbehälters, der flüssiges Konzentrat enthält, einen Behälter einzusetzen, in dem eine trockene Substanz in Pulverform enthalten ist, und diesen Behälter mit Wasser zu durchspülen, um das Konzentrat durch Lösen der trockenen Substanz zu erhalten. Dieses Konzentrat wird dann in die Dialysatleitung injiziert. Beispiele für die Erzeugung von Konzentrat durch Auflösen einer trockenen Substanz, die in einem Behälter enthalten ist, sind veröffentlicht in dem Patent EP 0 278 100 B1, der Patentanmeldung EP 0 475 825 A1, der Patentanmeldung EP 0 536 645 A2 und dem Patent DE 41 30 412 C1. In allen diesen Fällen ist die trockene Substanz in einem kartuschenförmigen Behälter enthalten, der am oberen und am unteren Ende jeweils einen Anschluß für einen ankommenden bzw. abgehenden Schlauch aufweist. Der Behälter enthält die trockene Substanz, z.B. Natriumhydrogenkarbonat oder Natriumchlorid, zur kontinuierlichen Herstellung von Dialysekonzentrat. Solche Behälter mit einer für die Behandlung ausreichenden Menge an trockener Substanz haben ein großes Gewicht, erfordern spezielle Halter zum Aufhängen, eine elektrische Abfrage und sind schwer zu handhaben. Außerdem ist die Entsorgung der Behälter, die in der Regel aus hartem Kunststoff bestehen, aufwendig.

Der Erfindung liegt die Aufgabe zugrunde, eine Dialysevorrichtung zu schaffen, bei der die Versorgung mit Trockensubstanz zur Erzeugung eines flüssigen Konzentrats erleichtert ist.

Die Lösung dieser Aufgabe erfolgt erfindungsgemäß mit den Merkmalen des Patentanspruchs 1.

Die Erfindung beruht auf der Erkenntnis, daß die für die Dialyse benötigten Substanzen, in der Regel Bikarbonat-Pulver (Natriumhydrogenkarbonat) leicht und schnell in eine gesättigte Lösung gebracht werden können. Überraschenderweise hat sich herausgestellt, daß für das Lösen solcher trockenen Substanzen ein starrer Behälter bzw. eine Kartusche nicht erforderlich ist. Vielmehr genügt ein flexibler Folienbeutel, in dem die trockene Substanz enthalten ist und der von Wasser durchströmt wird, um die Substanz zu lösen und als flüssiges Konzentrat abzuführen. Der flexible Folienbeutel hat im Vergleich zu Behältern oder Kartuschen ein viel geringeres Gewicht. Vorteilhaft ist ferner, daß der leere Folienbeutel nach Gebrauch flach zusammengelegt werden kann und dann nur ein Mindestmaß an Transport- und Lagervolumen benötigt. Auch die Entsorgung ist bei einem Folienbeutel wesentlich einfacher als bei einem voluminösen starren Behälter. Schließlich ist auch die Aufhängung des Folienbeutels vereinfacht.

Der Folienbeutel ist mit integrierten Zulauf- und Ablaufschläuchen versehen, an denen geeignete Schlauchverbinder angebracht sein können. Das Anschließen des Folienbeutels an den Wasserlieferanschluß und an den Konzentrat-Aufnahmeanschluß des Dialysegerätes erfordert kein Hantieren am Folienbeutel selbst, sondern nur an den jeweiligen Schläuchen. Dadurch ist die Gefahr eines Beutelbruchs verringert.

Durch die Abschrägung des Bodens des Folienbeutels zum Beutelauslaß hin wird der im Beutel verbleibende Salzrest verringert.

Der Folienbeutel kann auch eine Substanz enthalten, die in Wasser gelöst eine Desinfektionsmittellösung zum Desinfizieren der Schlauchleitungen des Dialysegerätes ergibt. Vorzugsweise ist der Beutel jedoch mit Natriumhydrogenkarbonat gefüllt.

Im folgenden wird ein Ausführungsbeispiel der Erfindung unter Bezugnahme auf die Zeichnung näher erläutert.

Es zeigen:
- Fig. 1: eine Ansicht des Folienbeutels, teilweise geschnitten,
- Fig. 2: den Anschluß des Folienbeutels an ein Dialysegerät, und
- Fig. 3: eine Teildarstellung von Fig. 2 zur Verdeutlichung des Spülbetriebes des Dialysegerätes bei abgenommenen Beutel.

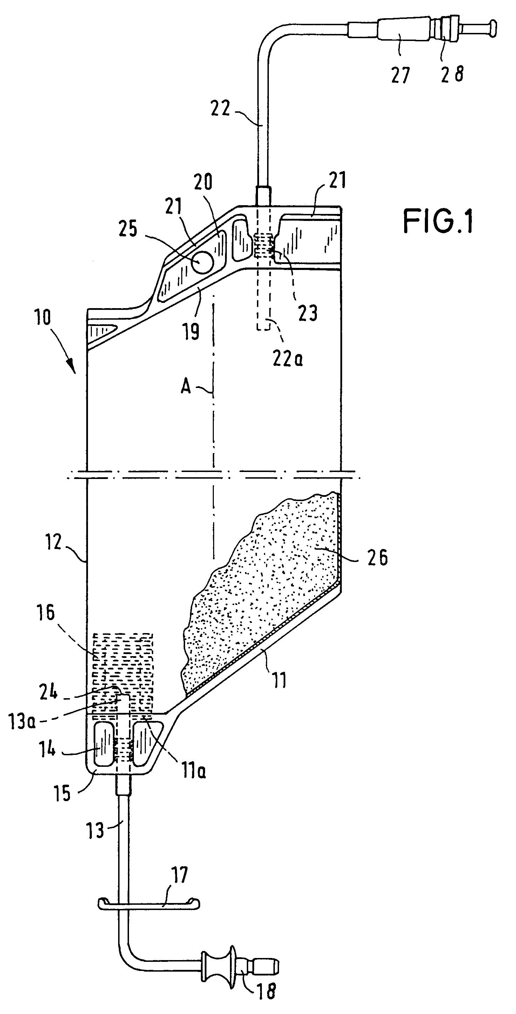

Der in Fig. 1 dargestellte Folienbeutel 10 besteht aus einem nahtlosen Folienschlauch aus Weichfolie, der an seinen Enden abgeschweißt ist, so daß der Folienbeutel allseitig geschlossen ist. Die Bodenschweißnaht 11 erstreckt sich schräg zur Längsrichtung des Schlauchs unter einem Winkel von etwa 30° über nahezu die gesamte Schlauchbreite. Am unteren Ende des Folienbeutels, nämlich an der tiefstgelegenen Spitze, die an die eine seitliche Faltlinie 12 des Folienschlauchs angrenzt, ist ein Ablaufschlauch 13 an dem Folienbeutel befestigt. Das Ende des Ablaufschlauchs 13 ist in einem Schlauchstutzen 13a eingeklebt, der sich durch einen nach außen abstehenden Beutelansatz 14 erstreckt, zwischen dessen Lagen er eingeschweißt ist. Der Beutelansatz 14 ist von einer umlaufenden Schweißnaht 15 begrenzt. Der Schlauchstutzen 13a ragt geringfügig hochstehend in das Innere des Beutels 10 und es ist dort von einem Filter 16 umgeben. Dieser Filter besteht aus einem gefalteten Flachbahnmaterial, das den Schlauchstutzen 13a haubenförmig umgibt und dessen unteres Ende in denjenigen Bereich 11a der Bodennaht 11 eingeschweißt ist, der den Schlauchstutzen 13a umgibt. Der Filter 16 ist ferner an den Schlauchstutzen 13a angeschweißt.

Auf dem Ablaufschlauch 13 sitzt außerhalb des Folienbeutels 10 eine Schlauchklemme 17 und das äußere Ende des Ablaufschlauchs 13 ist mit einem Schlauchverbinder 18 versehen.

Das obere Ende des Folienschlauchs ist mit einer schräg verlaufenden Quernaht 19 abgeschweißt, die etwa parallel zu der Bodennaht 11 verläuft. Das Folienmaterial bildet oberhalb der Quernaht 19 eine doppellagige Lasche 20, die durch Schweißnähte 21 begrenzt ist.

Der Zulaufschlauch 22 mündet in das obere Ende des Folienbeutels 10 und er erstreckt sich durch einen am Folienbeutel angeschweißten Schlauchstutzen 22a, der durch die Querschweißnaht 19 hindurchgeht und frei in das Innere des Folienbeutels hineinragt. Im Bereich der Lasche 20 ist der Schlauchstutzen 22a in einem Schweißabschnitt 23 zwischen die Lagen der Lasche 20 eingeschweißt. Der Zulaufschlauch 22 mündet an der höchsten Stelle des Folienbeutels 10 in diesen ein. Die Einmündungsstelle liegt rechts von der Längsmittelebene A des Folienschlauchs, also auf derjenigen Seite, die dem Beutelauslaß 24 entgegengesetzt ist.

Das äußere Ende des Zulaufschlauchs 22 ist mit einem Schlauchverbinder 27 versehen, der mit einem Verschlußstopfen 28 verschlossen werden kann.

In der Lasche 20 ist eine Aufhängeöse 25 ausgestanzt, die gegenüber der Längsmittelebene A zu der Seite des Beutelauslasses 24 hin geringfügig versetzt ist. Durch diese Anordnung der Aufhängeöse 25 wird erreicht, daß der gefüllte Beutel senkrecht hängt.

Der Folienbeutel 10 ist mit einer pulverförmigen trockenen Substanz 26, z.B. Natriumhydrogenkarbonat, gefüllt.

Der Beutel ist im wesentlichen parallelogrammförmig ausgebildet, wobei der Parallelogrammwinkel etwa 30° beträgt. Das Beutelmaterial ist eine Kunststoffolie V90, d.h. eine PP-PA-Verbundfolie, wobei die Innenschicht aus Polypropylen besteht. Das Filtergewebe des Filters 16 besteht ebenfalls aus PP. V90 als PP-PA-Verbundfolie hat sich als zweckmäßig erwiesen. Es können aber auch andere Kombinationen, z.B. EVA/EV0H, eingesetzt werden. Entsprechend ist das Filtermaterial, z.B. PP oder PA, auszuwählen. Der Filter besteht aus einer gefalteten Flachbahn mit einer effektiven Fläche von ca. 25 cm². In den Beutel sind jeweils die Schlauchstutzen 13a und 22a eingeschweißt. In die Schlauchstutzen sind die Schläuche 13 und 22 eingeklebt.

Der Beutel hat ein Volumen zur Aufnahme einer Befüllung von 800 g Bikarbonat. Es können auch andere Mengen vorkommen, vorzugsweise werden 600 g eingesetzt.

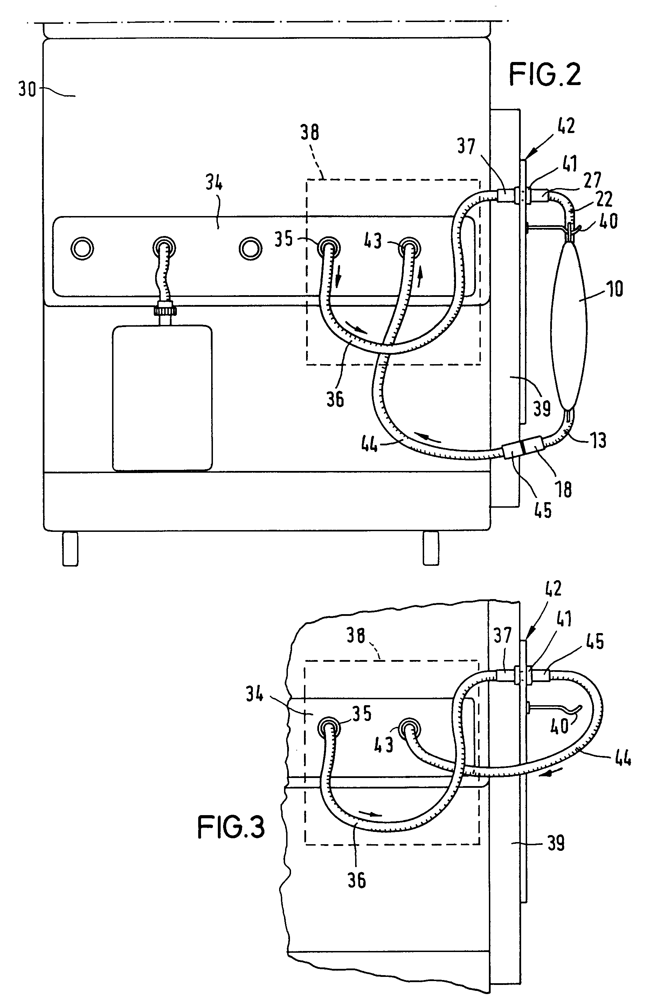

In Fig. 2 ist das Dialysegerät 30 dargestellt, das eine Anschlußleiste 34 für verschiedene Schläuche aufweist. An dieser Anschlußleiste 34 ist ein Wasserlieferanschluß 35 vorgesehen, an den ein Wasserschlauch 36 angeschlossen werden kann. Der Wasserschlauch ist an seinem externen Ende mit einem Schlauchverbinder 37 versehen. Der Wasserlieferanschluß 35 steht mit einem im Dialysegerät 30 enthaltenen Wasserbehälter 38 in Verbindung, der vorgewärmtes Wasser enthält.

Seitlich am Dialysegerät 30 ist ein Ständer 39 angebracht, der eine Aufhängevorrichtung 40 für den Folienbeutel 10 trägt. Oberhalb der Aufhängevorrichtung 40 ist am Ständer 39 eine Zweifach-Schlauchkupplung 41 montiert, in die der Schlauchverbinder 37 des Wasserschlauchs 36 eingesteckt ist. In das andere Ende der Zweifach-Schlauchkupplung 41 ist der Schlauchverbinder 27 des Zulaufschlauchs 22 des Folienbeutels 10 eingesteckt. Die Zweifach-Schlauchkupplung 41, die höher angeordnet ist als das obere Ende des Folienbeutels 10, bildet den höchstgelegenen Übergabepunkt 42. Dieser Übergabepunkt 42 ist etwa 20 mm höher angeordnet als der maximale Wasserstand des Wasserbehälters 38. Dadurch kann ein Auslaufen des Wasserbehälters 38 bei Fehlankopplung verhindert werden. Der Höhenunterschied sollte aber nicht zu groß sein, da sonst ein zu starker Unterdruck aufgebracht werden muß, um das Wasser aus dem Wasserbehälter 38 durch den Folienbeutel 10 zu ziehen.

An der Anschlußleiste 34 befindet sich ferner ein Konzentrat-Aufnahmeanschluß 43, der als Sauganschluß ausgebildet und mit einem Ansaugschlauch 44 verbunden ist. Der Ansaugschlauch 44 weist einen Schlauchverbinder 45 auf, der mit dem Schlauchverbinder 18 des Ablaufschlauchs 13 verbunden wird.

Beim Betrieb wird an dem Konzentrat-Aufnahmeanschluß 43 ein Sog erzeugt, wodurch Wasser durch den Folienbeutel 10 und die Leitungen 22 und 36 hindurch angesaugt wird. Das in den Folienbeutel 10 eintretende Wasser löst die trockene Substanz 26 auf, so daß ein Konzentrat gebildet wird, das durch die Leitungen 13 und 44 in das Dialysegerät eingeleitet wird. Das Durchströmen des Folienbeutels 10 mit Wasser erfolgt kontinuierlich mit geregelter Strömungsrate, die von einem (nicht dargestellten) Leitfähigkeitsmesser überwacht wird, der die Leitfähigkeit des den Folienbeutel 10 verlassenden Konzentrats mißt.

Wenn die trockene Substanz 26 aufgebraucht ist, wird der Ablaufschlauch 13 mit Hilfe der Schlauchklemme 17 abgequetscht, so daß das weitere Ansaugen von Konzentrat unterbleibt. Dann können die Schlauchverbinder 18 und 27 abgezogen werden, um den Folienbeutel 10 zu entfernen und zu entsorgen. Der noch im Beutel befindliche Flüssigkeitsinhalt kann abgelassen werden. Alternativ zum Ablassen des verbleibenden Flüssigkeitsinhaltes, der eine gebrauchsfähige Bikarbonatlösung darstellt, kann in einer bestimmten Zeit vor Ende der Dialyse durch automatisches Schließen eines Ventiles der Wasserzulauf unterbrochen und der Rest des Bikarbonatkonzentrates zur Bereitung der Dialyseflüssigkeit verbraucht werden. Dadurch besteht die Möglichkeit der Entsorgung des geleerten Beutels ohne jegliches Restvolumen.

Fig. 3 zeigt die Durchführung eines Desinfektions- oder Spülganges. Hierbei ist der Folienbeutel 10 von der Aufhängevorrichtung 40 abgenommen. Der Schlauchverbinder 37 des Wasserlieferanschlusses ist weiterhin mit der Zweifach-Schlauchkupplung 41 verbunden. In den anderen Anschluß der Zweifach-Schlauchkupplung 41 ist der Schlauchverbinder 45 des Saugschlauchs 44 eingesteckt. Auf diese Weise ist der Konzentrat-Aufnahmeanschluß 43 unmittelbar mit dem Wasserlieferanschluß 35 verbunden, so daß die Leitungen des Dialysegerätes mit Wasser durchspült werden können, dem ggf. eine Desinfektionslösung, zugegeben wurde.

Das Dialysegerät kann wahlweise mit dem beschriebenen Folienbeutel, der von Wasser durchströmt wird, oder mit einem Konzentratbehälter, der flüssiges Konzentrat enthält, betrieben werden. Wird flüssiges Konzentrat benutzt, dann wird der Wasserlieferanschluß 35 nicht verwendet.

## Patentansprüche

1. Dialysevorrichtung mit einem Dialysegerät (30) und einer an das Dialysegerät angeschlossenen Einrichtung zur Erzeugung eines flüssigen Konzentrats durch Lösen einer trockenen Substanz (26) in Wasser, wobei die trockene Substanz (26) in einem Behälter enthalten ist, der mit einem Wasserlieferanschluß (35) des Dialysegeräts (30) verbunden ist,
**dadurch gekennzeichnet,**
daß der die trockene Substanz (26) enthaltene Behälter aus einem flexiblen Folienbeutel (10) besteht, der einen zum Beutelauslaß (24) hin abfallenden Boden aufweist und an einem Ende mit einem Zulaufschlauch (22) und am Beutelauslaß (24) mit einem Ablaufschlauch (13) fest verbunden ist.

2. Dialysevorrichtung nach Anspruch 1, dadurch gekennzeichnet, daß der Folienbeutel (10) in flachliegendem Zustand im wesentlich parallelogrammförmig ausgebildet ist und daß der Zulaufschlauch (22) und der Ablaufschlauch (13) nahe den beiden Parallelogrammspitzen befestigt sind.

3. Dialysevorrichtung nach Anspruch 1 oder 2, dadurch gekennzeichnet, daß das beutelseitige Ende des Ablaufschlauchs (13) von einem Filter (16) umgeben ist.

4. Dialysevorrichtung nach Anspruch 3, dadurch gekennzeichnet, daß der Filter (16) ein Flachbahnfilter ist, der an den Folienbeutel (10) angeschweißt ist.

5. Dialysevorrichtung nach einem der Ansprüche 1 bis 4, dadurch gekennzeichnet, daß an dem Dialysegerät (30) eine Zweifach-Schlauchkupplung (41) befestigt ist, die einen von dem Wasserlieferanschluß (35) kommenden Wasserschlauch (36) mit dem Zulaufschlauch (22) des Folienbeutels (10) verbindet.

6. Dialysevorrichtung nach Anspruch 5, dadurch gekennzeichnet, daß der Schlauchverbinder (45) eines mit dem Konzentrat-Aufnahmeanschluß (43) verbundenen Ablaufschlauchs (44) in denselben Anschluß der Zweifach-Schlauchkupplung (41) paßt wie der Schlauchverbinder (27) des Zulaufschlauchs (22) des Folienbeutels (10).

7. Dialysevorrichtung nach einem der Ansprüche 1 bis 6, dadurch gekennzeichnet, daß der Folienbeutel (10) aus einem nahtlosen Folienschlauch besteht, der an seinen Enden durch schräg verlaufende Schweißnähte (11,19) abgeschweißt ist.

8. Dialysevorrichtung nach einem der Ansprüche 1 bis 7, dadurch gekennzeichnet, daß eine Aufhängeöse (25) von der Längsmittelebene (A) des Folienbeutels (10) zu derjenigen Seite versetzt ist, an der sich der Ablaufschlauch (13) befindet.

9. Dialysevorrichtung nach einem der Ansprüche 1 bis 8, dadurch gekennzeichnet, daß der Übergabepunkt (42) von dem Wasserschlauch (36) zu dem Zulaufschlauch (22) des Folienbeutels (10) höher liegt als der maximale Wasserstand eines Wasserbehälters (38) mit dem der Wasserlieferanschluß (35) verbunden ist.

10. Dialysevorrichtung nach einem der Ansprüche 1 bis 9, dadurch gekennzeichnet, daß das Beutelmaterial aus einer Folie mit guter Wasserdampf- und Gassperreigenschaft besteht.

11. Dialysevorrichtung nach einem der Ansprüche 1 bis 10, dadurch gekennzeichnet, daß das Beutelvolumen derart bemessen ist, daß die im Folienbeutel (10) enthaltene trockene Substanz (26) für die Zubereitung der für einen Dialysevorgang erforderlichen Konzentratmenge ausreicht.

12. Folienbeutel für eine Dialysevorrichtung nach einem der Ansprüche 1 bis 11.
